# EUROPÄISCHE PATENTANMELDUNG

(11) **EP 2 272 890 A1**
(43) Veröffentlichungstag der Anmeldung: **12.01.2011**
(21) Anmeldenummer: 10006572.1
(22) Anmeldetag: 24.06.2010
(51) Int. Cl.: C08G 64/24

(54) **Verfahren zur Herstellung von Polycarbonat**

(30) Priorität: 07.07.2009 DE 102009032020
(71) Anmelder: Bayer MaterialScience AG, 51368 Leverkusen (DE)
(72) Erfinder: Ooms, Pieter, Dr., 47800 Krefeld (DE); Bulan, Andreas, 40764 Langenfeld (DE); Rechner, Johann, Dr., 47906 Kempten (DE); Weber, Rainer, Dr., 51519 Odenthal (DE); Buts, Marc, 2570 Duffel (BE); Eynde, Johan Vanden, 9052 Zwijnaarde (BE)

(57) **Zusammenfassung**

Es wird ein kombiniertes Verfahren zur Herstellung von Polycarbonat aus Bisphenolen und Diarylcarbonaten, wobei das freigesetzte Monophenol wieder zur Herstellung des Diarylcarbonats und das bei der Herstellung des Diarylcarbonats entstehende Alkalihalogenid durch elektrochemische Oxidation, gegebenenfalls an einer Gasdiffusionselectrode, zu Chlor und Alkalilauge umgesetzt wird, wobei das Chlor zur Herstellung des Phosgens und die Alkalilauge zur Herstellung des Diarylcarbonats rückgeführt wird, beschrieben.

## Beschreibung

Die Erfindung betrifft ein kombiniertes Verfahren zur Herstellung von Polycarbonat aus Bisphenolen und Diarylcarbonaten, wobei das freigesetzte Monophenol mit Phosgen wieder zur Herstellung des Diarylcarbonats und das bei der Herstellung des Diarylcarbonats entstehende Alkalihalogenid durch elektrochemische Oxidation zu Chlor umgesetzt wird, wobei das Chlor zur Herstellung des Phosgens rückgeführt wird. Die ebenfalls gebildete Alkalilauge kann zur Herstellung des Diarylcarbonats wiederverwendet werden.

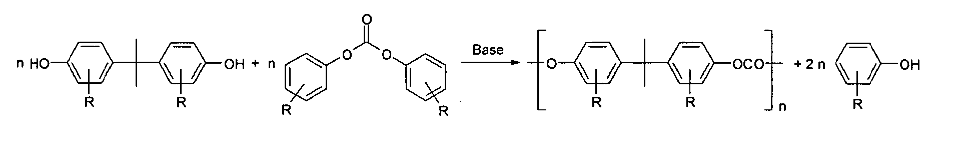

Die Herstellung aromatischer Polycarbonate nach dem Schmelzumesterungsverfahren ist bekannt und beispielsweise beschrieben in "Schnell", Chemistry and Physics of Polycarbonates, Polymer Reviews, Vol. 9, Interscience Publishers, New York, London, Sydney 1964, in D.C. Prevorsek, B.T. Debona and Y. Kersten, Corporate Research Center, Allied Chemical Corporation, Moristown, New Jersey 07960, "Synthesis of Poly(ester)carbonate Copolymers" in Journal of Polymer Science, Polymer Chemistry Edition, Vol. 19, 75-90 (1980), in D. Freitag, U. Grigo, P.R. Müller, N. Nouvertne, BAYER AG, "Polycarbonates" in Encyclopedia of Polymer Science and Engineering, Vol. 11, Second Edition, 1988, Seiten 648-718 und schließlich in Dres. U. Grigo, K. Kircher und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117-299.

In der WO 93/13084 A1 wird ein Prozess zur Herstellung von Polycarbonat beschrieben, bei dem das Monophenol, welches beim Umesterungsprozess freigesetzt wird, mit einem Carbonyldihalogenid (z.B. Phosgen) wiederum zum Diarylcarbonat umgesetzt werden kann. Bei diesem Verfahren wird das Carbonyldihalogenid aus CO und einer Metallhalogenidverbindung hergestellt. Diese Art der Herstellung über einen Katalysator ist aufwändig und unwirtschaftlich.

Die Herstellung der in dem Schmelzumesterungsverfahren für aromatische Polycarbonate eingesetzten Diarylcarbonate, z.B. durch den Phasengrenzflächenprozess, ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

In US 4,016,190 A wird ein Herstellungsverfahren von Diarylcarbonaten beschrieben, das bei Temperaturen >65°C betrieben wird. Der pH-Wert wird bei diesem Verfahren zunächst niedrig (pH 8 bis 9) und anschließend hoch (10 bis 11) eingestellt.

Optimierungen des Verfahrens durch Verbesserung der Durchmischung und Einhaltung eines engen Temperatur- und pH-Profils als auch Isolierung des Produkts werden in EP 1 219 589 A1, EP 1 216 981 A2, EP 1 216 982 A2 und EP 784 048 A1 beschrieben.

Bei diesen bekannten Verfahren macht jedoch ein hoher Restphenolwert im Abwasser dieser Prozesse, welche die Umwelt belasten können und die Klärwerke vor eine erhöhte Abwasserproblematik stellen, aufwändige Reinigungsoperationen erforderlich. So beschreibt WO 03070639 A1 eine Entfernung der organischen Verunreinigungen im Abwasser durch eine Extraktion mit Methylenchlorid.

Nach bekannten Verfahren wird die Natriumchlorid-haltige Lösung von Lösungsmitteln und organischen Resten befreit und dann entsorgt.

Gemäß WO 2000/078682 A1 oder US 6,340,736 A können die Natriumchlorid-haltigen Abwässer durch Ozonolyse gereinigt und dann bei der Natriumchlorid-Elektrolyse eingesetzt werden. Nachteil dieses Verfahrens ist die sehr kostenintensive Ozonolyse.

EP 1 894 914 A2 beschreibt die Rückführung von Natriumchlorid-haltigem Abwasser aus der Diarylcarbonatherstellung durch Phasengrenzflächenphosgenierung von Natriumphenolat (DPC) durch eine Chloralkali-Elektrolyse unter Erhöhung der Natriumchlorid-Konzentration durch Abtrennung des Reaktionsabwassers von den Waschphasen und erhöhten Wassertransport durch eine neue Membrantechnologie. Wiedereinsetzung von im Prozess entstehenden Monophenolen, Alkalilauge und Halogenid ist aber nicht beschrieben worden.

Ausgehend vom oben geschilderten Stand der Technik ist die Aufgabe, ein Polycarbonatherstellverfahren bereitzustellen, welches Produkte in hoher Reinheit und guter Ausbeute liefert und eine Reduzierung der Umweltbelastung bzw. Abwasserproblematik in den Klärwerken durch maximierte Rückführung von Nebenprodukten und Prozessabwasserlösungen, die aus der Polycarbonat-Produktion stammen, ermöglicht.

Weiterhin sollte bei dem Recycling die Umsetzung von Natriumchlorid zu Chlor und Natronlauge und gegebenenfalls Wasserstoff mit minimalem Energieeinsatz und daher Resourcen-schonend erfolgen.

Gegenstand der vorliegenden Erfindung ist daher ein Verfahren zur Herstellung von lösungsmittelfreiem Polycarbonat, ausgehend von Bisphenolen und Diarylcarbonaten, das **dadurch gekennzeichnet ist, dass** das Monophenol, das bei der Umsetzung zum Oligo-/Polycarbonat freigesetzt wird, wiederum zur Herstellung des aromatischen Diarylcarbonats wiedereingesetzt wird, wobei das bei der Herstellung des Diarylcarbonats entstehende Alkalihalogenid durch Elektrolyse in das Halogen und dieses wiederum nach Isolierung und Reaktion mit Kohlenmonoxid zum Carbonyldihalogenid umgesetzt wird, welches anschließend wieder mit der ebenfalls bei der Elektrolyse gebildeten Alkalilauge und dem Monophenol zur Herstellung des Diarylcarbonats eingesetzt wird.

Das erfindungsgemäße Gesamtverfahren ist daher flexibel, einfach in der Durchführung und liefert Produkte in hoher Reinheit, die für den Gesamtprozess außerordentlich wichtig sind unter gleichzeitiger Reduzierung der Umweltbelastung durch Wiedereinsetzung von im Prozess entstehenden Monophenolen, Alkalilauge und Halogenid.

Das erfindungsgemäße Verfahren umfasst wenigstens folgende Prozessschritte:
a) Umesterung eines oder mehrerer Bisphenole mit einem oder mehrere Diarylcarbonaten zum Oligo- / Polycarbonat und dem Monophenol,
b) Isolierung bzw. Trennung des Polycarbonats und des Monophenols,
c) Umsetzung des Monophenols in Anwesenheit von Alkalilauge mit dem Carbonyldihalogenid und Trennung der Produkte, wobei das Diarylcarbonat in den Schritt a) wiedereingesetzt wird,
d) Elektrochemische Oxidation des in Schritt c) erhaltenen Alkalihalogenids in Halogen und Alkalilauge,
e) Umsetzung wenigstens eines Teils des Halogens aus Schritt d) mit Kohlenmonoxid zum Carbonyldihalogenid, das wiederum wenigstens zum Teil in den Schritt c) eingesetzt wird,
f) Wiederverwendung wenigstens eines Teils der Alkalilauge aus d) im Schritt c).

Für das erfindungsgemäße Verfahren geeignete Bisphenole sind Dihydroxydiarylalkane der Formel (I),

HO-Z-OH (I)

worin Z ein divalenter organischer Rest mit 6 bis 30 Kohlenstoffatomen ist, der eine oder mehrere aromatische Gruppen enthält. Beispiele solcher Verbindungen, die in Schritt a) des erfindungsgemäßen Verfahrens eingesetzt werden können sind Dihydroxydiarylalkane wie Hydrochinon, Resorcin, Dihydroxydiphenyl, Bis-(hydroxyphenyl)-alkane, Bis(hydroxyphenyl)-cycloalkane, Bis-(hydroxyl-phenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren alkylierte, kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)propan (Bisphenol A (BPA)), 2,4-Bis-(4-hydroxyphenyl)-2-methylbutan, 1,3-Bis-[2-(4-hydroxyphenyl)-2-propyl]-benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan und 1,1-Bis-(4-hydroxyphenyl)-3,3,5-trimethylcyclohexan (Bisphenol TMC).

Besonders bevorzugte Bisphenole sind 4,4'-Dihydroxydiphenyl, 1,1-Bis-(4-Hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Geeignete Bisphenole sind z.B. in den US 2,999,835 A, US 3,148,172 A, US 2,991,273 A, US 3,271,367 A, US 4,982,014 A und US 2,999,846 A, in den deutschen Offenlegungsschriften DE 15 70 703 A, DE 20 63 050 A, DE 20 36 052 A, DE 22 11 956 A und DE 38 32 396 A, der französischen Patentschrift FR 1 561 518 A, in der Monographie von H. Schnell, Chemistry and Physics of Polycarbonates, Interscience Publishers, New York 1964, S. 28ff; S.102ffund von D.G. Legrand, J.T. Bendler, Handbook of Polycarbonate Science and Technology, Marcel Dekker New York 2000, S. 72ff. beschrieben.

Im Falle der erfindungsgemäßen Herstellung von Homopolycarbonaten wird nur ein Bisphenol eingesetzt, im Falle der erfindungsgemäßen Herstellung von Copolycarbonaten werden mehrere Bisphenole eingesetzt, wobei selbstverständlich die verwendeten Bisphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Es sei hier betont, dass das erfindungsgemäße Verfahren praktisch für alle bekannten Bisphenole eingesetzt werden kann.

Diarylcarbonate, die in Schritt a) des erfindungsgemäßen Verfahrens eingesetzt werden können, sind Di-C₆-C₁₄-Arylester, vorzugsweise die Diester von Phenol oder substituierten Phenolen, also Diphenylcarbonat oder z.B. Bissalicylcarbonat. Bezogen auf 1 Mol Diphenol werden die Diarylcarbonate in 1,01 bis 1,30 Mol, bevorzugt in 1,02 bis 1,15 Mol eingesetzt.

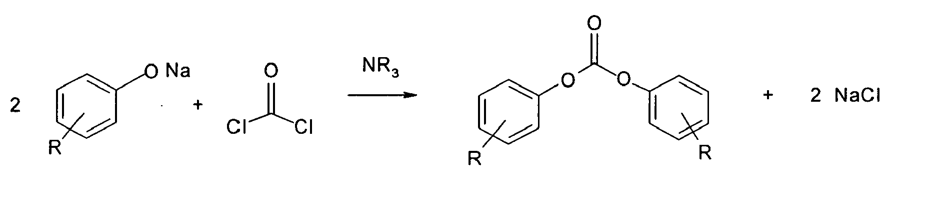

Das ausreagierte, höchstens noch Spuren an (< 2 ppm) Chlorkohlensäurearylestern enthaltende mindestens zweiphasige Reaktionsgemisch lässt man zur Phasentrennung absitzen. Die wässrige alkalische Phase (Reaktionsabwasser) wird abgetrennt und die organische Phase mit verdünnter Salzsäure und Wasser extrahiert. Die vereinigten Wasserphasen werden der Abwasseraufarbeitung zugeführt, wo Lösungsmittel- und Katalysatoranteile durch Strippen oder Extraktion abgetrennt und rückgeführt werden. Anschließend werden nach der Einstellung eines bestimmten pH-Wertes von z.B. 6 bis 8, z.B. durch Salzsäurezugabe, die gegebenenfallls noch verbleibenden organischen Verunreinigungen wie z.B. Monophenol durch Behandlung mit Aktivkohle entfernt und der Chloralkalielektrolyse zugeführt.

In einer anderen bevorzugten Variante der Aufarbeitung wird das Reaktionsabwasser nicht mit den Waschphasen vereinigt, aber nach Strippen oder Extraktion zur Abtrennung von Lösungsmitteln und Katalysatorresten, auf einem bestimmten pH-Wert von z.B. 6 bis 8, z.B. durch Salzsäurezugabe eingestellt und nach Abtrennung der noch verbleibenden organischen Verunreinigungen wie z.B. Monophenol durch Behandlung mit Aktivkohle der Chloralkalielektrolyse zugeführt.

Die Waschphasen können nach Entfernung der Lösungsmittel- und Katalysatoranteile durch Strippen oder Extraktion gegebenenfalls wieder der Synthese zugeführt werden.

Die Herstellung der Diarylcarbonate erfolgt in bekannter Weise durch Reaktion von Monophenolen und Phosgen in einem inerten Lösungsmittel in Gegenwart von Alkali und einem Stickstoffkatalysator nach dem Phasengrenzflächenverfahren unter Bildung des Alkalihalogenids (NaCl). Optimierungen des Verfahrens durch Verbesserung der Durchmischung und Einhaltung eines engen Temperatur- und pH-Profils als auch Isolierung des Diarylcarbonates werden in EP 1 219 589 A1, EP 1 216 981 A2, EP 1 216 982 A2 und EP 784 048 A1 beschrieben.

Besonders geeignete Monophenole zur Herstellung der Diarylcarbonate in Schritt c) sind Phenole der Formel (II) 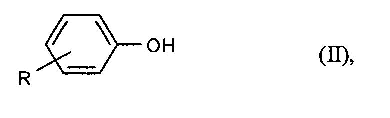 worin
- R: Wasserstoff, Halogen oder ein verzweigter oder unverzweigter C₁- bis C₉-Alkylrest oder Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Das eingesetzte Alkali zur Bildung des Phenolats kann eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.%-ige Lösung eingesetzt.

Die Reaktion b) kann durch Katalysatoren, wie tertiäre Amine, N-Alkylpiperidine oder Oniumsalze beschleunigt werden. Bevorzugt werden Tributylamin, Triethylamin und N-Ethylpiperidin verwendet.

Der eingesetzte Amin-Katalysator kann offenkettig oder zyklisch sein, besonders bevorzugt ist Triethylamin und Ethylpiperidin. Der Katalysator wird im neuen Verfahren vorzugsweise als 1 bis 55 Gew.%-ige Lösung eingesetzt.

Unter Oniumsalzen werden hier Verbindungen wie NR₄X verstanden, wobei R ein Alkyl und/oder Arylrest und/oder ein H sein kann und X ein Anion ist.

Phosgen kann in dem Verfahrensschritt b) flüssig, gasförmig oder gelöst in einem inerten Lösungsmittel eingesetzt werden.

In dem neuen Verfahren in Schritt b) bevorzugt verwendbare inerte organische Lösungsmittel sind beispielsweise Dichlormethan, Toluol, die verschiedenen Dichlorethane und Chlorpropanverbindungen, Chlorbenzol und Chlortoluol. Vorzugsweise wird Dichlormethan eingesetzt.

Die Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern. Bevorzugte Kettenabbrecher sind Phenol, Cumyl-phenol, Isooctylphenol, para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Beispiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenyl-methan, 2,2-Bis-[4,4-bis-(4-hydroxyphenyl)-cyclohexyl]-propan, 2,4-Bis-(4-hydroxyphenyl-isopropyl)-phenol, Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydro-indol und 1,1,1-Tri-(4-hydroxyphenyl)-ethan.

Die gegebenenfalls mitzuverwendenden 0,05 bis 2 Mol-%, bezogen auf eingesetzte Bisphenole, an Verzweigern, können mit den Bisphenolen zusammen eingesetzt werden.

Es ist darauf zu achten, dass die Reaktionskomponenten für den ersten Schritt, die Umesterung, also, die Bisphenole und die Diarylcarbonate frei von Alkali und Erdalkaliionen sind, wobei Mengen von kleiner 0,1 ppm an Alkali- und Erdalkaliionen toleriert werden können. Derart reine Bisphenole bzw. Diarylcarbonate sind erhältlich, indem man die Bisphenole bzw. Diarylcarbonate umkristallisiert, wäscht oder destilliert. Beim erfindungsgemäßen Verfahren soll der Gehalt an Alkali- und Erdalkalimetallionen sowohl im Bisphenol als auch im Diarylcarbonat einen Wert von < 0,1 ppm betragen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 - 190°C unter normalem Druck in 0 - 5 Stunden, bevorzugt 0,25 - 3 Stunden statt. Nach Zugabe des Katalysators wird durch Anlegen von Vakuum (bis zu 2 mm Hg) und Erhöhung der Temperatur (auf bis zu 260°C) durch Abdestillieren des Monophenols das Oligocarbonat aus dem Bisphenol und dem Diarylcarbonat hergestellt. Das so hergestellte Oligocarbonat hat eine mittlere Gewichtsmolmasse M_{w} (ermittelt durch Messung der rel. Lösungsviskosität in Dichlormethan oder in Mischungen gleicher Gewichtsmengen Phenol / o-Dichlorbenzol, geeicht durch Lichtstreuung) im Bereich von 2000 bis 18 000, bevorzugt von 4 000 bis 15 000. Hierbei wird die Hauptmenge an Monophenol (80 %) aus dem Prozess wiedergewonnen.

In der zweiten Stufe wird bei der Polykondensation durch weiteres Erhöhen der Temperatur auf 250 - 320°C, bevorzugt 270 - 295°C und einem Druck von < 2 mm Hg das Polycarbonat hergestellt. Hierbei wird der Rest an Monophenolen zurückgewonnen. Es können geringe Verluste an Monophenolen < 5 %, bevorzugt < 2 %, besonders bevorzugt < 1 % auftreten, verursacht durch Endgruppen im Polycarbonat und Restmonophenol im Polycarbonat. Diese Verluste müssen durch entsprechende Mengen an Monophenol für die Herstellung des Diarylcarbonats ausgeglichen werden.

Katalysatoren im Sinne des erfindungsgemäßen Verfahren für die Umesterung in Schritt a) sind alle anorganischen oder organischen basischen Verbindungen beispielsweise Lithium-, Natrium-, Kalium-, Cäsium-, Calzium-, Barium-, Magnesium-, -hydroxide, -carbonate, -halogenide, -phenolate, -diphenolate, -fluoride, -acetate, -phosphate, -hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetra-phenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-triazabicyclo-[4,4,0]-dec-5-en, 7,7'-Hexylidendi-1,5,7-triazabi-cyclo-[4,4,0]-dec-5-en, 7,7'-Decylidendi-1,5,7-triazabicyclo-[4,4,0]-dec-5-en, 7,7'-Dodecyliden-di-1,5,7-tri-aza-bicyclo-[4,4,0]-dec-5-en oder Phosphazene wie bei-spielsweise die Phosphazen-Base P₁-t-Oct = tert.-Octyl-imino-tris-(dimethylamino)-phosphoran, Phosphazen-Base P₁-t-Butyl = tert.-Butyl-imino-tris-(dimethylamino)-phosphoran, BEMP = 2-tert.-Butylimino-2-diethylamino-1,3-dimethyl-perhydro-1,3,2-diaza-2-phos-phoran.

Diese Katalysatoren werden in Mengen von 10⁻² bis 10⁻⁸ Mol, bezogen auf 1 Mol Bisphenol, eingesetzt.

Die Katalysatoren können auch in Kombination (zwei oder mehrere) miteinander eingesetzt werden.

Beim Einsatz von Alkali-/Erdalkali-Metallkatalysatoren kann es vorteilhaft sein, die Alkali-/ Erdalkali-Metallkatalysatoren zu einem späteren Zeitpunkt (z.B. nach der Oligocarbonatsynthese bei der Polykondensation in der zweiten Stufe) zuzusetzen. Die Zugabe des Alkali-/Erdalkali-Metallkatalysators kann z.B. als Feststoff oder als Lösung in Wasser, Phenol, Oligocarbonat oder Polycarbonat erfolgen.

Die Mitverwendung von Alkali- bzw. Erdalkali-Metallkatalysatoren widerspricht nicht der vorstehend erwähnten Forderung nach Reinheit der Reaktionspartner.

Die Reaktion des Bisphenols und des Diarylcarbonats zum Polycarbonat kann im Sinne des erfindungsgemäßen Verfahrens diskontinuierlich oder bevorzugt kontinuierlich durchgeführt werden, beispielsweise in Rührkesseln, Dünnschichtverdampfern, Fallflmverdampfern, Rührkesselkaskaden, Extrudern, Knetern, einfachen Scheibenreaktoren und Hochviskosscheibenreaktoren.

Die aromatischen Polycarbonate des erfindungsgemaßen Verfahrens sollen mittlere Gewichtsmolmassen M_{w} von 18000 bis 80000 vorzugsweise 19000 bis 50000 haben, ermittelt durch Messung der rel. Lösungsviskosität in Dichlormethan oder in Mischungen gleicher Gewichtsmengen Phenol / o-Dichlorbenzol, geeicht durch Lichtstreuung.

Es ist vorteilhaft, die aus der Umesterung des Bisphenols und des Diarylcarbonats zum Polycarbonat abgespaltenen und isolierten Monophenole vor dem Einsatz in die Diarylcarbonatsynthese aufzureinigen. Die Rohmonophenole, die beim Umesterungsprozeß isoliert werden, können je nach Umesterungsbedingungen und Destillationsbedingungen u.a. mit Diarylcarbonaten, dem Bisphenol, Salicylsäure, Isopropenylphenol, Phenoxybenzoesäurephenylester, Xanthon, dem Hydroxymonoarylcarbonat verunreinigt sein. Die Reinigung kann nach den üblichen Reinigungsverfahren, also z. B. Destillation oder Umkristallisation erfolgen. Die Reinheit der Monophenole liegt dann bei > 99 %, bevorzugt bei > 99,8 %, besonders bevorzugt bei > 99,95 %.

Die Herstellung von Phosgen aus Kohlenmonoxid und Chlor in Schritt a) ist an sich bekannt, beispielsweise aus EP 881 986 B1 oder EP 1 640 341 A. Die Umsetzung des Kohlenmonoxids erfolgt durch Umsetzung des Kohlenmonoxids mit Chlor zu Phosgen, zum Beispiel an einem Aktivkohle-Katalysator. Alternativkatalysatoren können aber auch eingesetzt werden. Hier kann auf den Stand der Technik verwiesen werden (z.B. DE 332 72 74; GB 583 477; WO 97/30932; WO 96/16898; US 6,713,035). Im technischen Maßstab wird Phosgen überwiegend durch Umsetzung von Kohlenmonoxid mit Chlor, bevorzugt an Aktivkohle als Katalysator hergestellt. Die stark exotherme Gasphasenreaktion erfolgt bei Temperaturen von mindestens 100°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkreislauf unter gleichzeitiger Nutzung der Reaktionswärme zur Dampferzeugung, wie z.B. in US 4,764,308 offenbart. Bevorzugt wird im Schritt a) Aktivkohle als Katalysator eingesetzt, wobei die Aktivkohle einen Gehalt an Gesamtschwefel von kleiner 1 Gew-%, insbesondere kleiner 0.5 Gew.-% bezogen auf das Gesamtgewicht des Katalysators enthält. Weiterhin wird der erfindungs-gemäße Schritt a) bevorzugt bei Temperaturen kleiner gleich 300°C durchgeführt und weiterhin wird der erfindungsgemäße Schritt a) mit der Erzeugung von Dampf kombiniert. Weiterhin bevorzugt hat der Katalysator nach Schritt a) eine spezifische Oberfläche von größer 10 m²/g.

Die Elektrolyse der Alkalichlorid-haltigen Lösung in Schritt d) kann direkt nach Abtrennung von Lösungsmittelresten und gegebenenfalls Katalysatorresten, insbesondere durch Extraktion oder Strippen der Lösung mit Wasserdampf, Neutralisierung, insbesondere mit Chlorwasserstoff oder Salzsäure, und Behandlung mit Adsorbentien, insbesondere mit Aktivkohle, erfolgen.

Üblicherweise werden z.B. zur Elektrolyse von Natriumchlorid-haltigen Lösungen Membranelektrolyseverfahren eingesetzt (siehe hierzu Peter Schmittinger, CHLORINE, Wiley-VCH Verlag, 2000). Die Elektrolyse kann gegebenenfalls mit erhöhtem Wassertransport und gegebenenfalls unter Verwendung einer Gasdiffusionselektrode als Kathode erfolgen, wie in DE 10 2006 041 465 A1 beschrieben.

Eine weitere bevorzugte Verfahrensvariante ist, dass dem Alkalichlorid-haltigen Abwasser aus c) vor der Elektrolyse d) durch ein Aufkonzentrierungsverfahren Wasser entzogen wird. Dies kann durch Eindampfung oder Membranverfahren erfolgen.

Gemäß der WO 01/38419 A kann die Natriumchlorid-haltige Lösung mittels thermischer Verfahren eingedampft werden, so dass eine hoch konzentrierte Natriumchlorid-Lösung der Elektrolysezelle zugeführt werden kann. Die Eindampfung ist jedoch energieintensiv und kostspielig.

Auch können beispielsweise die Umkehrosmose oder besonders bevorzugt die Membrandestillation oder Membrankontaktoren eingesetzt werden (siehe MELIN; RAUTENBACH, Membranverfahren; SPRINGER, BERLIN, 2003). Nachteilig hierbei ist der hohe Energiebedarf zur Überwindung der hohen osmotischen Drücke, wodurch die Wirtschaftlichkeit nicht mehr gegeben ist.

Besonders bevorzugt ist daher ein Verfahren, **dadurch gekennzeichnet, dass** die Alkalichloridhaltige Lösung aus c) vor der Elektrolyse d) mittels Membrandestillationsverfahren aufkonzentriert wird.

Die Aufkonzentrierung von NaCl-Lösungen durch osmotische Destillation ist energieschonend, insbesondere dann, wenn die aus der NaCl-Elektrolyse stammende NaOH-Lösung als Wasserakzeptor eingesetzt wird. Dies hat besonders dann Vorteile, wenn in der Diarylcarbonat-Produktion eine verdünnte Natronlauge eingesetzt wird, wobei dann zusätzlich das Wasser zur Verdünnung der Natronlauge eingespart werden kann.

Die osmotische Destillation erfolgt durch molekulare und gegebenenfalls Knudsen-Diffusion von Wasserdampf durch eine Membran. Dadurch ist die Diffusionsrate abhängig vom Unterschied zwischen den Wasserdampfdrücken auf beiden Seiten der Membran, sowie deren Porosität, Stärke und Gewundenheit.

Um eine effiziente Aufkonzentrierung zu ermöglichen, sollte als Wasser-Akzeptor eine konzentrierte Lösung eines Alkalihydroxids, bevorzugt Natrium- oder Kaliumhydroxid, besonders bevorzugt Natriumhydroxid verwendet werden.

Durch Kombination vom Betrieb der Elektrolysezellen und Aufkonzentrierungsverfahren kann das Natriumchlorid aus dem Abwasser der Diphenylcarbonat-Herstellung nahezu quantitativ wiedergewonnen werden.

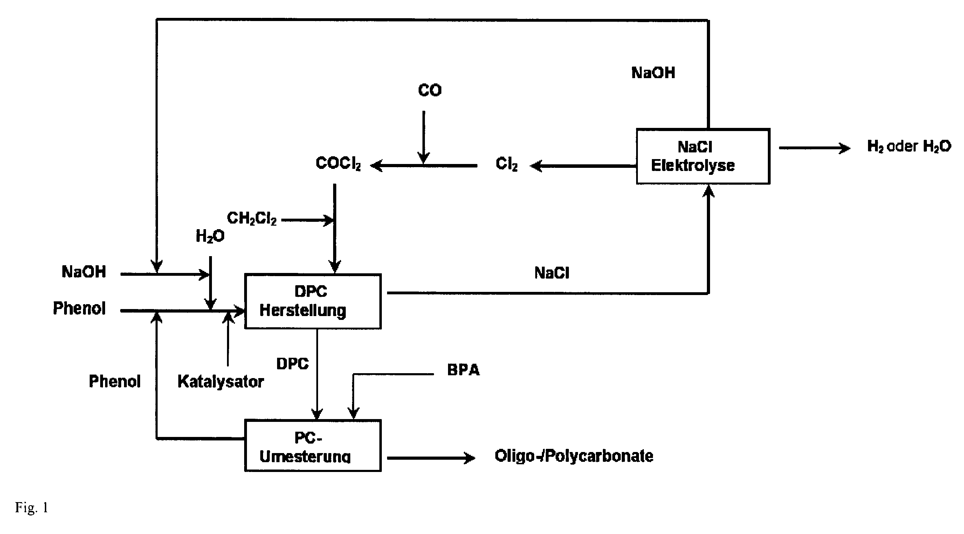

Fig. 1 zeigt schematisch ein kombiniertes Verfahren zur Herstellung von Polycarbonat aus Bisphenol A und Diphenylcarbonat, wobei das abgetrennte Phenol mit Phosgen wieder zur Herstellung des Diphenylcarbonats und das bei der Herstellung des Diphenylcarbonats entstehende Natriumchlorid durch elektrochemische Oxidation zu Chlor umgesetzt wird und das Chlor zur Herstellung des Phosgens zurückgeführt wird. Die ebenfalls anfallende Alkalilauge kann zur Herstellung des Diphenylcarbonats wiederverwendet werden. Im Falle einer Aufkonzentrierung der Natriumchlorid-haltigen Abwasserlösung vor der Elektrolyse durch osmotische Destillation mit einer konzentrierten Natronlauge als Wasserakzeptor kann zusätzliches Wasser zur Verdünnung der Natronlauge für die Diphenylcarbonatherstellung wiederverwendet werden.

Die Beispiele sollen das erfindungsgemäße Verfahren anhand der Polycarbonatherstellung unter Rückführung des Phenols in die Herstellung von Diphenylcarbonat und Elektrolyse der dabei anfallenden Natriumchlorid-haltigen Abwasserphasen in Chlor und Natronlauge, die wieder für das Diphenylcarbonat-Herstellungsverfahren verwendet werden können, darstellen.

### Beispiele

### Beispiele 1

### a) Herstellung von Polycarbonat

Aus einer Vorlage werden 8.600 kg/h Schmelzegemisch, bestehend aus 4.425 kg/h Diphenylcarbonat (20.658 mol/h) und 4.175 kg/h Bisphenol A (18.287 mol/h), unter Zusetzen von 0,52 kg des Phenoladdukts von Tetraphenylphosphoniumphenolat mit 65,5 % Tetraphenylphosphoniumphenolat/h (0,786 mol/h; das sind 0,0043 Mol-%) gelöst in 4,5 kg Phenol/h durch einen Wärmetauscher gepumpt, auf 190°C erwärmt und durch eine Verweilkolonne bei 12 bar und 190°C geführt. Die mittlere Verweilzeit beträgt 50 Minuten.

Die Schmelze wird dann über ein Entspannungsventil in einen unter 200 mbar stehenden Abscheider geleitet. Die abfließende Schmelze wird in einem, ebenfalls unter 200 mbar stehenden, Fallfilmverdampfer wieder auf 189°C erwärmt und in einer Vorlage aufgefangen. Nach einer Verweilzeit von 20 Minuten wird die Schmelze in die nächsten drei, gleichartig aufgebauten Stufen gepumpt. Die Bedingungen in der 2. / 3. / 4. Stufe sind 100/74/40 mbar, 218 / 251 / 276°C und 20 Minuten / 10 Minuten / 10 Minuten. Das entstandene Oligomere hat eine relative Viskosität von 1,09. Alle Brüden werden über Druckregelungen in eine unter Vakuum stehende Kolonne geführt und als Kondensate abgeleitet.

Danach wird das Oligomer in einem sich anschliessenden Korbreaktor bei 278°C und 3,0 mbar bei einer Verweilzeit von 45 Minuten zu einem höhermolekularen Produkt aufkondensiert. Die relative Viskosität beträgt 1,195. Die Brüden werden kondensiert.

Vom Schmelzestrom, der in einen weiteren Korbreaktor geleitet wird, wird mittels einer Zahnradpumpe ein Teilstrom von 150 kg/h Schmelze abgezweigt, mit 185 g/h einer 5 %-igen wässrigen Phosphorsäure versetzt, über einen statischen Mischer mit einem Länge-zu-DurchmesserVerhältnis von 20 gerührt und wieder in den Hauptschmelzestrom zurückgeleitet. Direkt nach dem Zusammentreffen wird die Phosphorsäure im gesamten Schmelzestrom mittels eines weiteren statischen Mischers homogen verteilt.

Die so behandelte Schmelze wird in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden werden in der Vakuumanlage und dahinter kondensiert.

Das erhaltene Polycarbonat hat folgende Kennzahlen: relative Viskosität 1,201 / phenolische OH 255 [ppm] / DPC 71 [ppm] / BPA 6 [ppm] / Phenol 56 [ppm]. Das abdestillierte Phenol kann wieder in die Diphenylcarbonatherstellung rückgeführt werden.

### b) Herstellung und Aufarbeitung von Diphenylcarbonat

In einen senkrecht stehenden, gekühlten Rohrreaktor wurde kontinuierlich ein Gemisch aus 145,2 kg/h 14,5 %-iger Natronlauge, hergestellt durch Verdünnung von 65,8 kg/h einer 32,0 %-igen Natronlauge mit 79,4 kg/h vollentsalztem Wasser (VE-Wasser), und 48,3 kg/h frisches und/oder zurückgewonnenes Phenol mit einer Lösung von 86,2 kg/h Methylenchlorid und 27,5 kg/h Phosgen (8 Mol % Überschuss bzgl. auf Phenol) zusammengebracht. Dieses Reaktionsgemisch wurde auf eine Temperatur von 33°C gekühlt und nach einer mittleren Verweilzeit von 15 Sekunden ein pH-Wert von 11,5 gemessen. Zu diesem Reaktionsgemisch wurden nun in der zweiten Stufe des Verfahrens 5,4 kg/h 50,0 %-iger NaOH zudosiert, so dass der pH-Wert der zweiten Reaktionsstufe nach einer weiteren Verweilzeit von 5 Minuten 8,5 beträgt. In der zweiten Stufe des Verfahrens wurde das Reaktionsgemisch durch das Durchleiten eines mit Verengungen versehenen Rohres ständig gemischt. Die Reaktionstemperatur wurde nach erneuter Zugabe der NaOH durch Kühlen auf 30°C eingestellt. Nach Abtrennung der organischen von der wässrigen Phase (Reaktionsabwasser) wurde die DPC-Lösung mit 0,6 %-iger Salzsäure und Wasser gewaschen. Nach Entfernung des Lösungs-mittels wurde 99,9 %-iges Diphenylcarbonat erhalten. Das Reaktionsabwasser wurde nicht mit den Waschphasen vereinigt und wurde durch Strippen mit Wasserdampf von Lösungsmittelresten und Katalysator befreit. Nach Neutralisierung (pH 7) mit Salzsäure und Behandlung mit Aktivkohle enthielt das Reaktionsabwasser 17,0 % NaCl und < 2 ppm Phenol.

Das Abwasser konnte ohne weitere Reinigung der elektrochemischen Oxidation zugeführt werden.

### c) Elektrochemische Oxidation des Reaktionsabwassers aus b)

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,6 Gew.%-ige NaCl-Lösung entnommen werden. Der aus der Anodenkammer entnommenen NaCl-Lösung wurden 0,133 kg/h an 17,0 Gew.%-igen Reaktionsabwasser aus der Diphenylcarbonatherstellung aus Beispiel 1b) und 0,0655 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.% NaOH, die aus der Kathodenseite entnommene Natronlauge hatte eine Konzentration von 33,0 % NaOH. 0,180 kg/h der 33,0 %-igen Lauge wurden dem Volumenstrom entnommen, der Rest wurde mit 0,060 kg/h Wasser aufgestockt und wieder in das Kathodenelement zurückgeführt.

25,8 % des umgesetzten Natriumchlorids stammten aus dem DPC-Reaktionsabwasser.

### Beispiel 2

### a) Herstellung von Polycarbonat

Das Polycarbonat wurde wie in 1a) beschrieben hergestellt.

### b) Herstellung und Aufarbeitung von Diphenylcarbonat

Es wurde verfahren wie in Beispiel 1b) beschrieben, nur wurde das Reaktionsabwasser mit den Waschphasen zu Gesamtprozessabwasser vereinigt und durch Strippen mit Wasserdampf von Lösungsmittelresten und Katalysator befreit. Nach Neutralisierung mit Salzsäure und Behandlung mit Aktivkohle enthielt das Gesamtprozessabwasser 13,0 % NaCl und < 2 ppm Phenol.

Das Abwasser konnte ohne weitere Reinigung der elektrochemischen Oxidation zugeführt werden.

### c) Elektrochemische Oxidation des Gesamtprozessabwassers aus 2b)

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.% NaCl. Aus der Anodenkammer konnte eine 18,6 Gew.%-ige NaCl-Lösung entnommen werden. Der aus der Anodenkammer entnommenen NaCl-Lösung wurden 0,127 kg/h an 13,0 Gew.%-igen Reaktionsabwasser aus der Diphenylcarbonatherstellung aus Beispiel 2b) und 0,0717 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.% NaOH, die aus der Kathodenseite entnommene Natronlauge hatte eine Konzentration von 33,0 % NaOH. 0,180 kg/h der 33,0 %-igen Lauge wurden dem Volumenstrom entnommen, der Rest wurde mit 0,060 kg/h Wasser aufgestockt und wieder in das Kathodenelement zurückgeführt.

18,8 % des umgesetzten Natriumchlorids stammten aus dem DPC-Gesamtprozessabwasser.

### Beispiel 3

### a) Herstellung von Polycarbonat

Das Polycarbonat wurde wie in 1a) beschrieben hergestellt.

### b) Herstellung von Diphenylcarbonat

Nach dem in 1b) beschriebenen Verfahren wurde das Diphenylcarbonat hergestellt und das Reaktionsabwasser abgetrennt und behandelt. Das Reaktionsabwasser konnte ohne weitere Reinigung der elektrochemischen Oxidation zugeführt werden.

### c) Elektrochemische Oxidation des Reaktionsabwassers mit Gasdiffusionselektroden

Da für die Herstellung von DPC kein Wasserstoff benötigt wird, kann auf die Bildung von Wasserstoff bei der Elektrolyse verzichtet werden. Die Elektrolyse wurde daher mit Gasdiffusionselektroden betrieben. Die Stromdichte betrug 4 kA/m², Temperatur Auslauf Kathodenseite 88°C, Temperatur Auslauf Anodenseite 89 °C. Eingesetzt wurde eine Elektrolysezelle mit Standard Anoden-Coating der Fa. DENORA, Deutschland. Eingesetzt wurde eine Ionenaustauschermembran der Fa.DuPont, Nafion 982 WX. Die Elektrolysespannung betrug 2,11 V. Die Natriumchlorid-Konzentration der der Anodenkammer entnommenen Lösung betrug 17,0 Gew.% an NaCl. Der aus der Anodenkammer entnommenen NaCl-Lösung wurden 0,178 kg/h an 17,0 Gew.%-igem Reaktionsabwasser und 0,0579 kg/h festes Natriumchlorid zugegeben. Die Lösung wurde anschließend wieder in die Anodenkammer eingespeist. Der Wassertransport über die Membran betrug 4,9 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.% NaOH, die aus der Kathodenseite entnommene Natronlauge hatte eine Konzentration von 31,5 Gew-.% NaOH. 0,189 kg/h der 31,5 %-igen Lauge wurden dem Volumenstrom entnommen, der Rest wurde mit 0,0312 kg/h Wasser aufgestockt und wieder in das Kathodenelement zurückgeführt.

Der Anteil an umgesetztem Natriumchlorid aus dem DPC-Reaktionsabwasser betrug 34,4 %.

## Patentansprüche

1. Verfahren zur Herstellung eines Polycarbonats, umfassend wenigstens folgende Schritte:
a) Umesterung eines oder mehrerer Bisphenole mit einem oder mehrtere Diarylcarbonate zum Oligo- / Polycarbonat und dem Monophenol,
b) Isolierung bzw. Trennung des Polycarbonats und des Monophenols,
c) Umsetzung des Monophenols in Anwesenheit von Alkalilauge mit dem Carbonyldihalogenid und Trennung der Produkte, wobei das Diarylcarbonat in den Schritt a) wiedereingesetzt wird,
d) Elektrochemische Oxidation des in Schritt c) erhaltenen Alkalihalogenids in Halogen und Alkalilauge,
e) Umsetzung wenigstens eines Teils des Halogens aus Schritt d) mit Kohlenmonoxid zum Carbonyldihalogenid, das wiederum wenigstens zum Teil in den Schritt c) eingesetzt wird,
f) Wiederverwendung wenigstens eines Teils der Alkalilauge aus d) im Schritt c).

2. Verfahren gemäß Anspruch 1, **dadurch gekennzeichnet, dass** als Bisphenole in Schritt a) Dihydroxydiarylalkane der Formel (I)
HO-Z-OH (I),
worin Z ein divalenter organischer Rest mit 6 bis 30 Kohlenstoffatomen ist, der eine oder mehrere aromatische Gruppen enthält, verwendet werden.

3. Verfahren gemäß Anspruch 1 oder 2, **dadurch gekennzeichnet, dass** als Diarylcarbonate in Schritt a) Di-C₆-C₁₄-Arylester verwendet werden.

4. Verfahren gemäß einem der Ansprüche 1 bis 3, **dadurch gekennzeichnet, dass** als Monophenole zur Herstellung der Diarylcarbonate in Schritt c) Phenole der Formel (II) 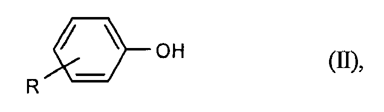 verwendet werden, worin
R Wasserstoff, Halogen oder ein verzweigter oder unverzweigter C₁- bis C₉-Alkylrest oder Alkoxycarbonylrest ist.

5. Verfahren gemäß einem der Ansprüche 1 bis 4, **dadurch gekennzeichnet, dass** das eingesetzte Diarylcarbonat Diphenylcarbonat ist, das Bisphenol Bisphenol A ist, das Carbonyldihalogenid Phosgen ist, die Alkalilauge Natronlauge ist.

6. Verfahren gemäß Anspruch 5, **dadurch gekennzeichnet, dass** die Elektrolyse des Natriumchlorids aus c) zu Natronlauge und Chlor unter Einsatz von Gasdiffusionselektroden als Kathode erfolgt.

7. Verfahren gemäß einem der Ansprüche 1 bis 6, **dadurch gekennzeichnet, dass** das Alkalihalogenid-haltige Abwasser aus Schritt c) vor der Elektrolyse gemäß Schritt d) von Lösungsmittelresten und gegebenenfalls Katalysatorresten, insbesondere durch Extraktion oder Strippen der Lösung mit Wasserdampf, Neutralisierung, insbesondere mit Chlorwasserstoff oder Salzsäure, und/oder Behandlung mit Adsorbentien, insbesondere mit Aktivkohle abgetrennt wird.

8. Verfahren gemäß einem der Ansprüche 1 bis 7, **dadurch gekennzeichnet, dass** das Alkalihalogenid-haltige Abwasser aus Schritt c) vor der Elektrolyse gemäß Schritt d) aufkonzentriert wird.

9. Verfahren gemäß Anspruch 8, **dadurch gekennzeichnet, dass** das Alkalihalogenid-haltige Abwasser aus Schritt c) vor der Elektrolyse gemäß Schritt d) mittels osmotischer Destillation mit Natronlauge als Wasserakzeptor aufkonzentriert wird.
